# EUROPEAN PATENT SPECIFICATION

(11) **EP 0 467 007 B1**
(45) Date of publication and mention of the grant of the patent: **17.04.1996**
(21) Application number: 90870113.9
(22) Date of filing: 18.07.1990
(51) Int. Cl.: C07C 15/02

(54) **Transalkylation process**
Transalkylierungsverfahren
Procédé de transalkylation

(43) Date of publication of application: 22.01.1992
(73) Proprietor: FINA TECHNOLOGY, INC., Dallas, Texas 75206 (US)
(72) Inventor: Butler, James R., Houston, Texas (US)
(74) Representative: Leyder, Francis

(56) References cited:
- DE-A- 3 437 615
- US-A- 4 169 111
- US-A- 4 459 426
- US-A- 4 555 311

## Description

### FIELD OF THE INVENTION

This invention relates to the transalkylation of polyalkylated aromatic compounds and more particularly to alkylation-transalkylation processes involving alkylation of a benzene feed stock with a C₂-C₄ alkylating agent and liquid phase transalkylation of resulting polyalkylbenzenes, treatment of the alkylation product in a separation zone, and recycle of at least a portion of the transalkylation product to the separation zone.

### BACKGROUND OF THE INVENTION

Processes for the alkylation of aromatic feedstocks and the use of zeolite molecular sieve catalysts in aromatic alkylation processes are well known in the art. Such alkylation processes may be carried out in the vapor phase, in the liquid phase, or under conditions in which both liquid and vapor phases exist.

An example of vapor phase alkylation is found in U.S. Patent No. 4,107,224 to Dwyer. Here, vapor phase ethylation of benzene over a zeolite catalyst is accomplished in a down flow reactor. The output from the reactor is passed to a separation system in which ethylbenzene product is recovered, with the recycle of polyethylbenzenes to the alkylation reactor where they undergo transalkylation reactions with benzene.

An example of an alkylation-transalkylation process in which the output from the alkylation reaction zone is passed directly to the transalkylation zone is disclosed in U.S. Patent No. 3,551,510 to Pollitzer et al. In the Pollitzer process, alkylation is carried out using an alkylating agent, characterized as an olefin acting compound, over a solid phosphoric acid alkylation catalyst. The olefin acting compound may be selected from materials such as monoolefins, diolefins, polyolefins, actylenic hydrocarbons, alkyl halides, alcohols, ethers and esters. The output from the alkylation reaction zone, which includes polyethylbenzenes, is supplied to a transalkylation reaction zone along with an aromatic substrate, e.g., benzene. The transalkylation zone is loaded with an acid extracted crystalline aluminosilicate catalyst, specifically mordenite, and is operated in a upflow mode. Exemplary transalkylation conditions including a liquid hourly space velocity of 1.0, a pressure of 3447 kPa (500 psig) and a temperature of 250°C. The output from the transalkylation zone is supplied to a separation zone from which a polyalkylaromatic, e.g., polyethylbenzene, is withdrawn and recycled to the alkylation reaction zone.

Another alkylation transalkylation process is disclosed in U.S. Patent No. 4,008,290 to Ward. Ward, like the patent to Pollitzer, discloses the use of a solid phosphoric acid catalyst in the alkylation zone. In the Ward process, benzene is reacted with propylene to produce cumene. The output from the alkylation reactor in Ward is split so that a portion, containing principally benzene and cumene, is recycled to the alkylation reactor. Another portion containing principally benzene, cumene, propane and di-and tri-isopropylbenzene is supplied to a separation zone. In the separation zone a di- and tri-isopropylbenzene rich stream is separated and supplied to a transalkylation zone along with benzene. The transalkylation zone also contains a solid phosphoric acid catalyst. A cumene rich effluent is withdrawn from the transalkylation zone and recycled to the separation zone.

U.S. Patent No. 4,169,111 to Wight discloses an alkylation-transalkylation process for the manufacture of ethylbenzene employing crystalline aluminosilicates in the alkylation and transalkylation reactors. The catalysts in the alkylation and transalkylation reactors may be the same or different and include low sodium content zeolites, preferably less than 0.5 weight percent Na₂0, having silica/alumina mole ratios between 2 and 80 and preferably between 4-12. Exemplary zeolites include molecular sieves of the X, Y, L, B, ZSM-5 and Omega crystal types with steam stabilized Y zeolite containing about 0.2% Na₂0 being preferred. The alkylation reactor is operated in a down flow mode and under temperature and pressure conditions in which some liquid phase is present. The transalkylation reactor, which is described as generally requiring higher temperatures than the optimum temperature for alkylation in order to achieve maximum transalkylation efficiency, is also operated in a down flow mode. In the Wight procedure, the output from the alkylation reactor is cooled and supplied to a benzene column from which benzene is recovered overhead and recycled to the alkylation reactor. The bottoms fraction from the benzene column is supplied to an ethylbenzene column from which ethylbenzene is recovered as the process product. The bottoms product from the ethylbenzene column is supplied to a third column which is operated to provide a substantially pure diethylbenzene overhead fraction which contains from 10 to 90% preferably 20 to 60% of the total diethylbenzene feed to the column. The diethylbenzene overhead fraction is recycled to the alkylation reactor while a side cut containing the remaining diethylbenzene and triethylbenzene and higher molecular weight compounds is supplied to the transalkylation reactor along with benzene. The effluent from the transalkylation reactor is recycled to the benzene column.

U.S. Patent No. 4,774,377 to Barger et al. discloses an alkylation/transalkylation process which, like the above-described Wight process, involves the use of separate alkylation and transalkylation reaction zones, with recycle of the transalkylated product to an intermediate separation zone. In the Barger process, the temperature and pressure conditions are adjusted so that the alkylation and transalkylation reactions take place in essentially the liquid phase. The transalkylation catalyst is an aluminosilicate molecular sieve including X-type, Y-type, ultrastable-Y, L-type, omega type and mordenite type zeolites with the latter being preferred. The catalyst employed in the alkylation reaction zone is a solid phosphoric acid containing material. Aluminosilicate alkylation catalysts may also be employed and water varying from 0.01 to 6 volume percent is supplied to the alkylation reaction zone. The output from the alkylation reaction zone is supplied to first and second separation zones. In the second reaction zone intermediate aromatic products and trialkylaromatic and heavier products are separated to provide an input to the transalkylation reaction zone having only dialkyl aromatic components, or diethylbenzene in the case of an ethylbenzene manufacturing procedure or diisopropylbenzene in the case of cumene production. A benzene substrate is also supplied to the transalkylation zone for the transalkylation reaction and the output from the transalkylation zone is recycled to the first separation zone. The alkylation and transalkylation zones may be operated in a downflow, upflow or horizontal flow configurations.

### SUMMARY OF THE INVENTION

In accordance with the present invention there is provided an alkylation-transalkylation process involving alkylation of an aromatic substrate with a C₂-C₄ alkylating agent coupled with separation to recover a monoalkylated aromatic product and liquid phase transalkylation of a polyalkylated product. In one aspect of the invention, both the alkylation and transalkylation reactions are carried out in the liquid phase over molecular sieve aromatic alkylation and transalkylation catalysts. The output from the alkylation reaction zone is supplied to a separation zone which is operated to produce a lower boiling fraction comprising the aromatic substrate, which may be recycled to the alkylation reaction zone, and a higher boiling fraction comprising a mixture of monoalkylated and polyalkylated aromatics. The higher boiling fraction is supplied to a second separation zone to produce a second lower boiling fraction comprising the desired monoalkylated product and a higher boiling fraction comprising polyalkylated product.

At least a portion of the polyalkylated fraction including substantially all dialkylated and trialkylated aromatics is supplied, along with the aromatic substrate, to a transalkylation reaction zone containing a molecular sieve transalkylation catalyst. The transalkylation zone is operated under liquid phase conditions to cause disproportionation of the polyalkylated fraction to arrive at a disproportionation product having a reduced polyalkylated aromatic content and an enhanced monoalkylated aromatic content. At least a portion of the disproportionation product is supplied to the first separation zone. In a specific application of the invention directed to the production of ethylbenzene or cumene, the output from the transalkylation zone is supplied to a third separation zone from which benzene and a monoalkyl benzene fraction (ethylbenzene or cumene) is recovered and recycled to the separation zone.

In another embodiment of the invention, a benzene feed stock and a C₂-C₄ alkylating agent are supplied to an alkylation reaction zone containing a molecular sieve alkylation catalyst and which is operated to produce an alkylated product comprising a mixture of monoalkyl and polyalkyl benzenes. In this embodiment of the invention the alkylation zone may be operated under liquid phase or vapor phase conditions with the output from the alkylation zone being subjected to separation steps as described above. The transalkylation reaction zone is operated at an average temperature below the average temperature of the alkylation reaction zone and under conditions to maintain the benzene in the liquid phase. In a specific application of this embodiment of the invention to a procedure employing vapor phase ethylation of benzene followed by liquid phase transalkylation, the average temperature of the transalkylation reaction zone is at least 100°C less than the average temperature of the alkylation reaction zone.

In yet a further aspect of the invention involving the alkylation of a benzene feed stock with a C₂-C₄ alkylating agent, the alkylation catalyst is selected from the group consisting of zeolite beta, zeolite omega, and zeolite Y and the alkylation reactor is operated under conditions to maintain the benzene feed stock in the liquid phase as described previously. The effluent from the alkylation reactor is subjected to separation steps along the lines described above and subsequent to separation to recover the desired monoalkylbenzene product, e.g., ethylbenzene or cumene, at least a portion of the polyalkylbenzene fraction including substantially all of the dialkylbenzene content and a predominant portion of the trialkylbenzene content is supplied to the transalkylation zone containing a transalkylation catalyst selected from a group consisting of a zeolite Y and zeolite omega. Preferably the alkylation catalyst comprises zeolite beta. It is also preferred that the transalkylation catalyst comprise zeolite omega.

In a further embodiment of the invention directed specifically to the production of ethylbenzene, in which the alkylation reaction takes place over an aromatic alkylation catalyst selected from the group consisting of zeolite beta and zeolite omega, the output from the alkylation reaction zone is supplied to a benzene separation zone. A higher boiling fraction comprising an ethylbenzene/polyethylbenzene mixtures is supplied from the benzene separation zone to an ethylbenzene separation zone. This zone is operated to produce a lower boiling product fraction comprising ethylbenzene and a higher boiling fraction comprising polyethylbenzene containing no more than 5 wt. % ethylbenzene. The polyethylbenzene fraction is supplied along with benzene to a transalkylation reaction zone which preferably contains a transalkylation catalyst selected from the group consisting of zeolite Y and zeolite omega.

### BRIEF DESCRIPTION OF THE DRAWINGS

FIGURES 1a-1c and 2a-2c are graphs illustrating the results of transalkylation experiments carried out using two different zeolite Y catalysts.

FIGURES 3a-3c are graphs illustrating the results of experimental work carried out in the transalkylation of diethylbenzene using zeolite omega.

FIGURES 4a-4c are a series of graphs showing experimental work carried out with a rare earth zeolite.

FIGURES 5a, 5b and 6 are graphs illustrating further experimental work employing a zeolite Y catalyst.

FIGURE 7 is a simplified schematic flow diagram illustrating one embodiment of the invention in which a polyethylbenzene fraction is subjected to a residue extraction step prior to transalkylation.

FIGURE 8 is a schematic illustration of a modification of the process of FIGURE 7 in which the output from the transalkylation reactor is subjected to a separation step prior to recycle.

FIGURE 9 is a simplified schematic illustration of yet another embodiment of the invention in which the bottoms fraction from an ethylbenzene column is supplied directly to a transalkylation reactor with the output of the transalkylation reactor being supplied to a downstream separation zone.

FIGURE 10 is a schematic flow diagram showing a modification of the embodiment of FIGURE 9.

### DETAILED DESCRIPTION

The preferred application of the invention involves liquid phase alkylation over a molecular sieve alkylation catalyst selected from the group consisting of zeolite beta and zeolite omega coupled with liquid phase transalkylation over a molecular sieve transalkylating catalyst selected from the group consisting of zeolite Y and zeolite omega. An especially preferred embodiment of the invention involves the use of zeolite beta as an alkylation catalyst and zeolite omega as a transalkylation catalyst. However, as will appear below, other molecular sieve catalysts can be employed in carrying out the present invention. Moreover, while a preferred application of the invention is in the use of liquid-phase transalkylation in conjunction with liquid-phase alkylation, the invention can be carried out employing vapor-phase alkylation, as disclosed, for example, in the aforementioned patent to Dwyer, coupled with liquid-phase transalkylation and appropriate recycle of the transalkylated product to a separation zone.

In its more general aspects, the invention involves transalkylation coupled with aromatic alkylation employing C₂-C₄ alkylating agents which, broadly stated, can be alkylating agents of the type disclosed in the aforementioned patent to Pollitzer et al., such as olefins, alkynes, alkyl halides, alcohols, ethers and esters. The most widely used alkylating agents are ethylene and propylene applied in the production of ethylbenzene and cumene, respectively. The invention is especially applicable to the ethylation of benzene under conditions in a manner in which byproduct xylene yields are reduced and the invention will be described specifically by reference to the production of ethylbenzene together with the attendant transalkylation of polyethylbenzenes.

As noted previously, a conventional process for the production of ethylbenzene involves recycling polyethylbenzenes, separated from the ethylbenzene product, to the alkylation reactor where they undergo transalkylation to yield ethylbenzene. A byproduct of this procedure is increased xylene yield in the effluent from the alkylation reactor. The presence of xylenes complicates downstream processing and separation steps. A particular impact of a significant xylene content in the product stream is that it often mandates operation of the distillation column from which the ethylbenzene is taken overhead in a manner to provide a substantial ethylbenzene content, oftentimes 15-20% or more, in the bottom polyethylbenzene fraction. For example, ethylbenzene produced in accordance with the present invention can be employed in the production of styrene by catalytic dehydrogenation. The boiling points of ortho xylene and styrene are very close, within 1°C of one another. As a practical matter, the ethylbenzene specifications will call for a very low xylene content, normally less than 2000 ppm. In order to meet this specification, it is normally necessary to operate the ethylbenzene column under moderate distillation conditions resulting in a high ethylbenzene content in the bottoms fraction as described above. The present invention, by carrying out polyethylbenzene transalkylation in a separate reactor under relatively mild liquid phase conditions, minimizes the xylene make in the manufacturing process. This enables ethylbenzene recirculation to be reduced by limiting the ethylbenzene content in the polyethylbenzene fraction to 5 wt.% or less and, where preferred catalysts are used to further minimize xylene make, down to about 2 wt.% or less ethylbenzene.

A preferred aspect of the present invention involves supplying the polyethylbenzene fraction, including both diethylbenzene and the triethylbenzene and higher molecular weight compounds to the transalkylation reactor as contrasted with separating out a substantial portion of the diethylbenzene for recycle to the alkylation zone, as disclosed in the aforementioned patent to Wight, or separating out trialkylaromatics with transalkylation only of dialkylbenzene, as disclosed in the aforementioned patent to Barger. In this respect, depending upon the configuration of the interface of the transalkylation reactor and polyethylbenzene or other separation zones, substantially all of the diethylbenzene and substantially all or most of the triethylbenzene content will be supplied to the transalkylation reactor. In either case, the practical effect of this embodiment of the invention is that recycle to the alkylation reactor is limited to benzene and lighter components, e.g., ethylene, while most, if not all of the triethylbenzenes together with diethylbenzenes are retained in the system ultimately for conversion to benzene and ethylbenzene. This offers significant advantages over the prior art processes, not only in terms of reduced xylene make as described previously, but also in terms of ultimate product yield.

In the transalkylation zone the LHSV (liquid hourly space velocity) based upon benzene and alkylbenzenes is less than the LHSV in the primary reaction zone based upon benzene - preferably less than one half thereof - the value itself being within the range 1-10.

In experimental work relative to the invention a number of catalysts were employed in transalkylation tests carried out in an upflow, flooded-bed reactor, that is, only a liquid phase was in contact with the catalyst. The feed employed in this experimental work was an approximate 1:1 mixture of benzene and the polyethylbenzene overheads fraction from a commercial operation employing vapor-phase alkylation of benzene to produce ethylbenzene. A typical feed employed in the experimental work had the composition as shown below in Table I.

**TABLE I**

| Component | Wt.% |
|---|---|
| Non-aromatics | 0.032 |
| Benzene | 50.241 |
| Toluene | 0.000 |
| Ethylbenzene | 6.117 |
| p + M-Xylene | 0.000 |
| Styrene | 0.063 |
| o-Xylene | 0.066 |
| Cumene | 3.973 |
| n Propylbenzene | 7.816 |
| m + p Ethyltoluene | 2.053 |
| 1,3,5-Trimethylbenzene | 0.128 |
| o-Ethyltoluene | 0.356 |
| 1,2,4-Trimethylbenzene | 0.536 |
| 1,2,3-Trimethylbenzene | 0.401 |
| m-Diethylbenzene | 14.808 |
| o + p-Diethylbenzene | 7.328 |
| Butylbenzenes | 1.653 |
| Heavies | 4.429 |

In the experimental work, the average pressure was about 2068 kPa (300 psia) with a pressure drop across the reactor ranging from about 34 to 103 kPa (5 to 15 psi). The temperature profile across the reactor was relatively constant with an endotherm from the inlet to the outlet of less than 10°C and usually less than 5°C. The experimental runs were initiated at relatively low temperatures, usually less than 100°C and progressively increased as described later. The space velocity was maintained relatively constant at a value of 6 hr⁻¹ (LHSV) based on the total hydrocarbon feed. Diethylbenzene conversions and selectivity to ethylbenzene were measured as a function of catalyst age (duration of the run) along with the production of various other components including xylenes.

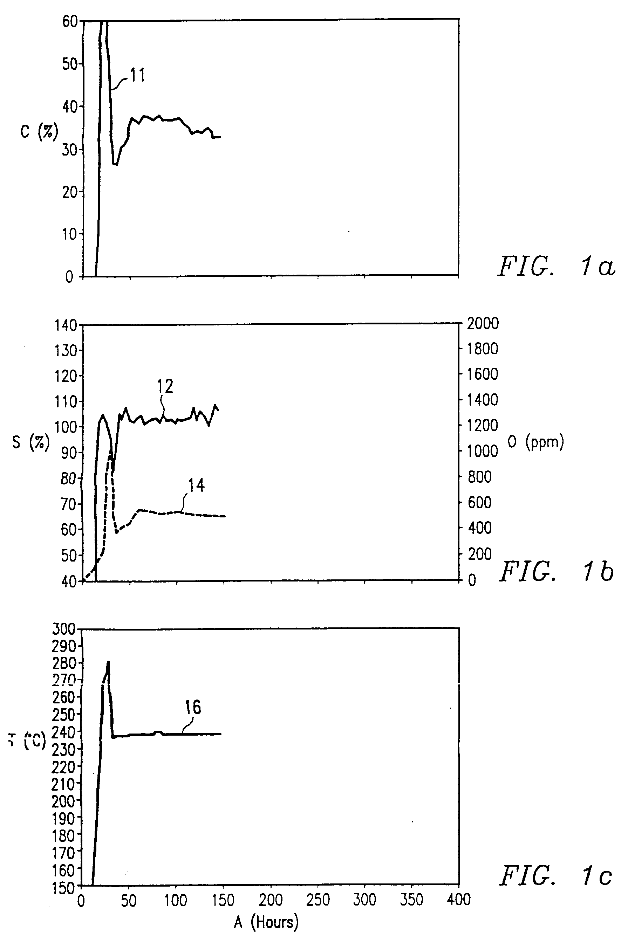

In a first test run, the catalyst used was a commercially available zeolite Y (identified herein as Catalyst A) in which the inlet temperature was progressively increased up to about 235°C and stabilized there with an average temperature increase through the reactor of only 1° or 2°C. The results of this experimental work are illustrated in FIGURES 1a-1c in which percent diethylbenzene conversion C, percent selectivity to ethylbenzene, S, ortho xylene production O, in ppm, and temperature, T, °C are plotted as curves 11, 12, 14 and 16, respectively versus the catalyst age A, in hours, on the abscissa. As can be seen from an examination of the data presented in FIGURE 1, the diethylbenzene conversion stabilized in about the 32-37% range for a reactor temperature of about 237°C with the catalyst showing very little deactivation over the duration of the run. The selectivity to ethylbenzene was virtually 100%. During the run, O-xylene production stabilized at about 400 to 500 ppm.

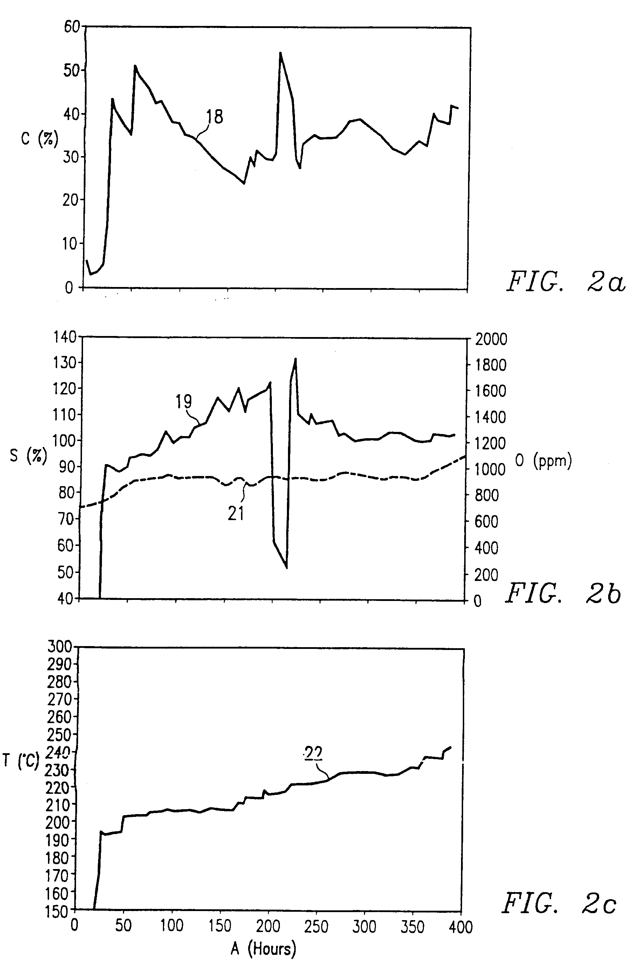

Another test run was carried out using an experimental zeolite Y identified herein as catalyst B. The results of this run are set forth in FIGURES 2a-2C in which curves 18, 19, 21 and 22 are graphs of diethylbenzene conversion, C, selectivity to ethylbenzene, S, parts per million o-xylene, O, and temperature, T, respectively plotted as a function of catalyst Age A. In this experiment, the catalyst was run for nearly 400 hours with the temperature, after initialization, increasing slightly with time to a final value of about 240°C. As can be seen diethylbenzene conversion was relatively good, mostly in the 30-40% range at relatively moderate temperatures. Selectivity to ethylbenzene was greater than 90% and during most of the run was virtually at 100%. The o-xylene content of the product stream stabilized at about 900 ppm.

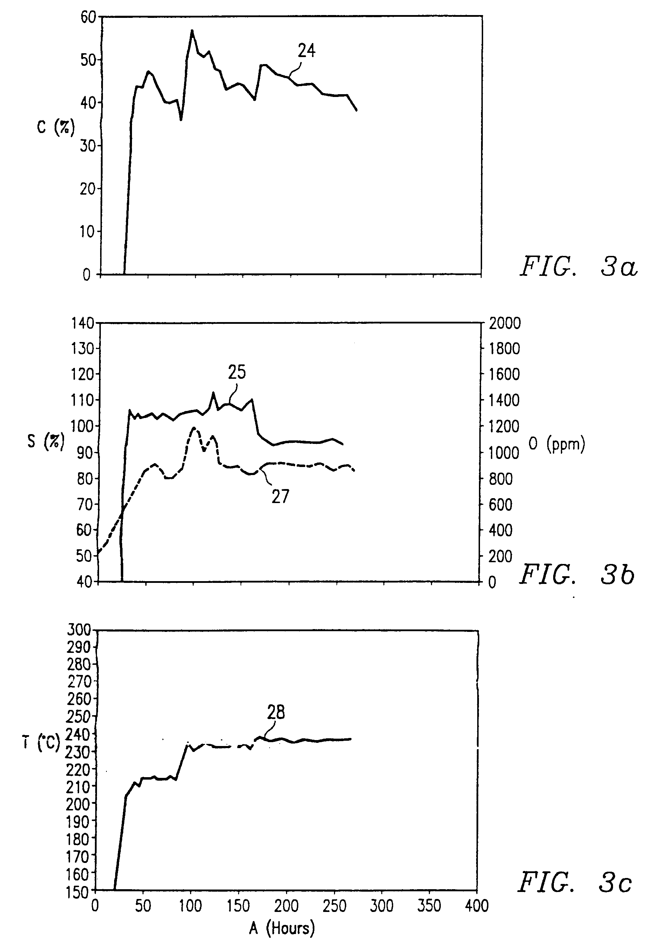

Yet another test run was carried out employing a zeolite omega catalyst identified herein as catalyst C. The results here in terms of diethylbenzene conversion, selectivity and as a function of time and temperature are set forth in FIGURES 3a-3c. In FIGURE 3 curves 24, 25, 27 and 28 are graphs of diethylbenzene conversion, selectivity to ethylbenzene, o-xylene content (ppm), O, and temperature, T, °C as a function of catalyst age on the abscissa. As shown in FIGURE 3, diethylbenzene conversion was, on balance, slightly better than for catalysts A and B, and fell generally into the 40-50% range at reactor temperatures ranging from about 210° to about 236°C. Selectivity to ethylbenzene was more than 90% over much of the run at virtually 100%. O-xylene content stabilized at about 800-900 ppm. The catalyst showed very little deactivation over the life of the run.

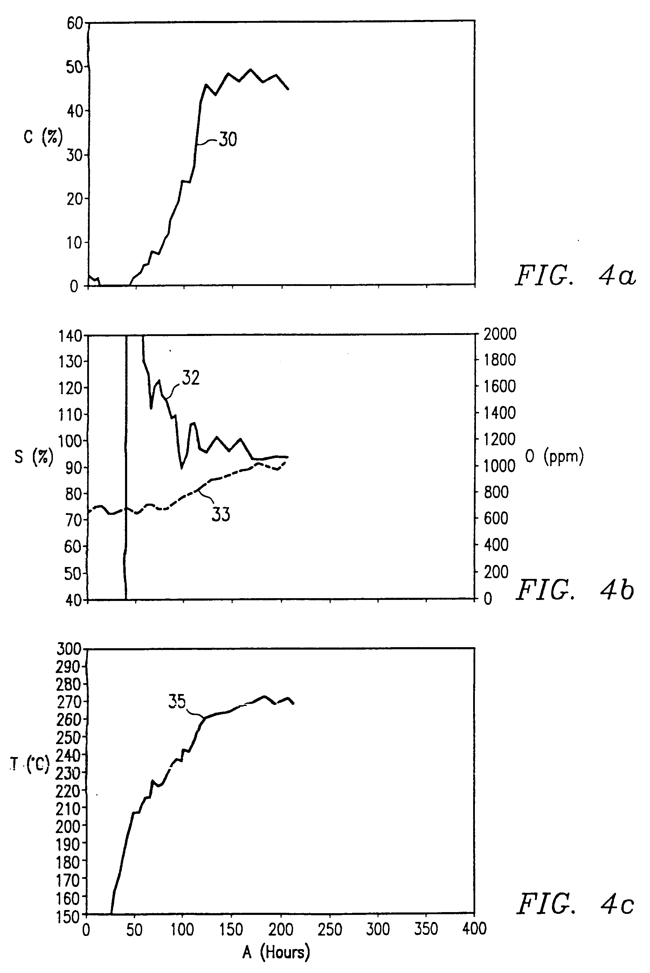

A rare earth zeolite Y identified herein as catalyst D was employed in yet another test. The results for catalyst D are set forth in FIGURES 4a-4c with curves 30, 32, 33 and 35 representing graphs of diethylbenzene conversion, selectivity to ethylbenzene, ppm o-xylene and temperature, respectively, as a function of catalyst age. Catalyst D showed relatively good results including diethylbenzene conversion in the 40-50% range. Initial selectivity was about 100%, with selectivity falling off slightly to about 90% toward the end of the run. While good conversion and selectivity were achieved, the reaction temperature was substantially higher than for the zeolites Y and omega, rising to about 270°C at the conclusion of the run, about 210 hours.

The feeds for the experimental work depicted in FIGURES 1-4 conformed generally to the composition shown in Table I. However, the feed for the first test run (catalyst A) was free of ortho xylene and the feed for the second run (catalyst B) contained about 0.02% para and meta xylene.

Additional experimental work under the above-identified conditions were carried out employing three additional catalysts; catalyst E, a cation exchange resin available from Rohm and Haas under the designation Amberlyst 15, catalyst F, a superacidic alumina available from Harshaw-Filtrol under the designation 3998 and catalyst G a nickel modified mordenite available from Union Carbide under the designation Ni-Cn9040. Catalyst E showed little diethylbenzene conversion and no ethylbenzene production up to the time the experiment was terminated, at about 50 hours and a temperature of 155°C, due to experimental difficulties. Catalyst F produced diethylbenzene conversions ranging from about 10 to 20% at temperatures ranging from about 300°-450°C with selectivity to ethylbenzene for the most part being less than 50%. Catalyst G was run for 100 hours at temperatures ranging up to 350°C and showed almost no diethylbenzene conversion.

The zeolite Y catalysts identified above as Catalyst A and B were also used in down flow trickle bed reactors where a substantial gas phase was present. Fresh and regenerated catalysts were used. This experimental work was carried out at pressures of about 2275 kPa (330 psig), nominal space velocities of about 10hr⁻¹ (LHSV) and average reactor temperatures of about 300°C in the case of fresh catalyst A, about 300°-400°C in the case of fresh catalyst B and about 200°C in the case of the regenerated catalysts. For fresh catalyst A, initial diethylbenzene conversion was about 24% but this fell off rapidly after a few hours. The catalyst was then regenerated and under the less severe temperature conditions of about 200°C, initial diethylbenzene conversion was high, about 60% but this, again, reduced to only a few percent after about 24 hours.

When employing fresh catalyst B the initial diethylbenzene conversion was over 50%, but this fell to about 20% after about 5 hours and then decreased further to only a few percent. The regenerated catalyst B, when run at the lower temperature of about 200°C, showed an initial diethylbenzene conversion of about 58% which declined to about 27% after 29 hours, at which time the run was terminated.

Yet additional experimental work was carried out employing the zeolite Y identified above as catalyst B in which the feed was a relatively pure diethylbenzene mixed in approximately equal parts with benzene. Unlike the feed stock employed in the experiment work of FIGURES 1 through 4, the pure diethylbenzene feed stock contained only very small amounts of material susceptible to cracking or other conversion reactions, e.g., deethylation, to produce xylenes and was also free of xylenes. The make up of the feed stock in this experimental work is set forth below in Table II.

**TABLE II**

| Components | Wt.% |
|---|---|
| Non Aromatics | 0.01 |
| Benzene | 56.58 |
| Toluene | 0.09 |
| Ethylbenzene | 0.01 |
| Xylenes | 0.0000 |
| n-PR-BZ | 0.02 |
| m,p-ethyltoluene | 0.03 |
| o-ethyltoluene | 0.01 |
| 124 trimethylbenzene | |
| sec-BU-BZ | 0.47 |
| 123 Trimethylbenzene | |
| m,Diethylbenzene | 27.62 |
| o,p-diethylbenzene | 14.27 |
| n-BU-BZ | 0.35 |
| Heavies | 0.54 |

In this test run, the inlet and outlet pressures were held at 2137 and 2103 kPa (310 and 305 psig), respectively. The average temperature of the reactor was increased approximately linearly with time from an initial value of about 198° to a final value of about 298°C. The space velocity was generally held within the range of about 5.8-6.0hr⁻¹ (LHSV) with the exception of about two-thirds of the way through the test where it fell to about 5.1 before recovering to the higher value.

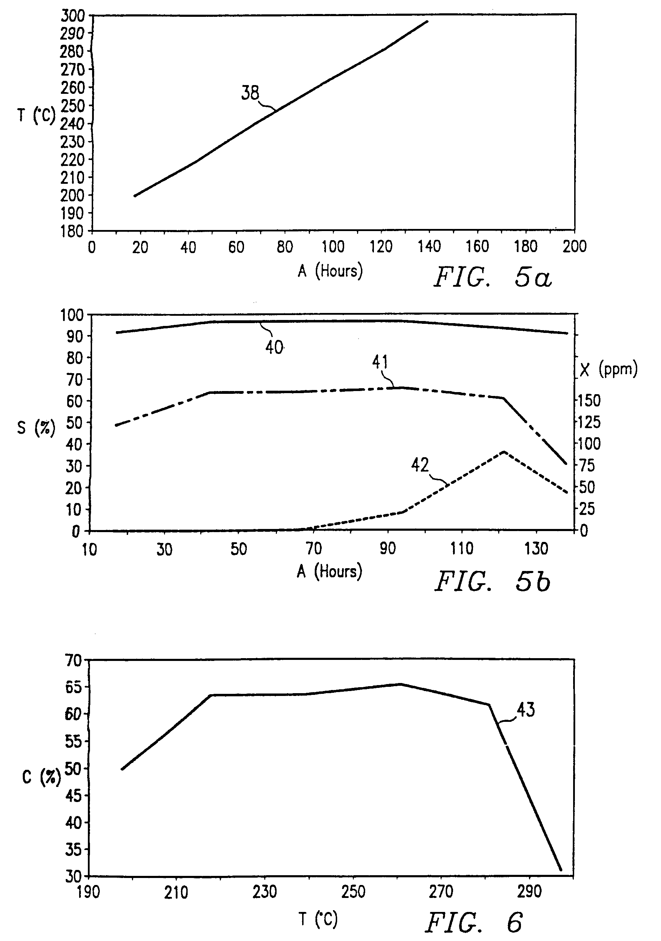

The results of this test run are set forth in FIGURES 5 and 6. In FIGURE 5a, curve 38 is a graph of temperature, T, versus catalyst age A in hours on the abscissa. In FIGURE 5b curves 40 and 41 are graphs of percent selectivity to ethylbenzene and percent ethylbenzene conversion, respectively. Curve 42 is a graph of the total xylene make, X, expressed in ppm, based upon the amount of ethylbenzene produced. FIGURE 6, shows the relationship between ethylbenzene conversion and temperature. Curve 43 is a graph of ethylbenzene conversion, C, on the ordinate versus temperature, T, on the abscissa.

As indicated by the data set forth in FIGURE 5, xylene make remained low throughout the test run. No xylene was produced until the temperature was increased to about 260°C (which generally corresponded to the reduction in space velocity to about 5.1 hours⁻¹ as reported previously). Percent conversion remained good until the temperature was increased above 280°C. As indicated in FIGURE 6, ethylbenzene conversion appears to remain above 50% over a temperature range of about 200°-290°C with the optimum range appearing to be about 210° to 280°C.

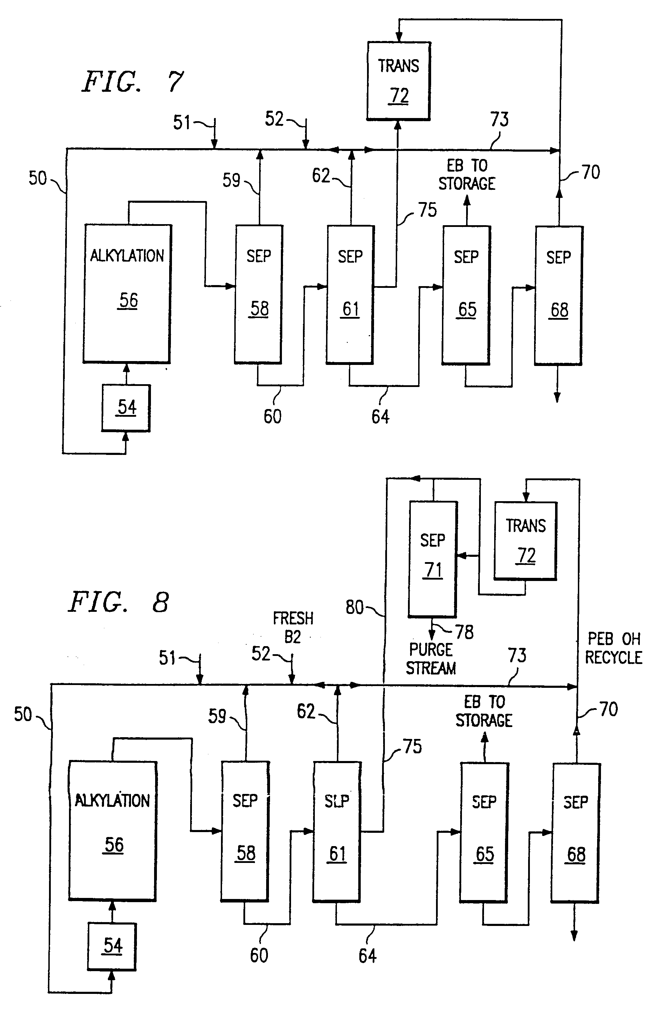

With further reference to the drawings, FIGURE 7 through 10 illustrate schematic flow diagrams illustrating different embodiments of the invention. It will be assumed for purposes of discussion that the invention is applied in the production of ethylbenzene by reaction of ethylene with benzene and that the alkylation reaction as carried out in a flooded-bed liquid-phase alkylation reactor employing zeolite beta, zeolite Y or zeolite omega as the alkylation catalyst. However, as noted previously and as discussed in greater detail below, the alkylation step can be conducted as a vapor- phase reaction employing a catalyst such as silicalite or ZSM-5.

Referring first to FIGURE 7, a feed stream 50 containing ethylene and benzene supplied via lines 51 and 52, respectively, is passed first to a dehydrator 54, where the water content is reduced to a level of about 100 ppm or less, preferably about 50 ppm or less, and then to an alkylation reaction zone 56. The alkylation reactor which may comprise a plurality of series connected adiabatic reactors with interstage injection of ethylene and also interstage cooling, normally will be operated at an average temperature of about 220°C and under sufficient pressure, about 4137 kPa (600 psia) or above, to maintain the benzene in the liquid phase and at least about 2 mole percent of ethylene solublized in the benzene. As an alternative to using adiabatic reactors, one or more isothermal reactors can be employed with suitable cooling means used to maintain a substantially constant temperature (little or no temperature differential) from the inlet to the outlet of the reactor. The effluent stream from the alkylation reactor is supplied to a prefractionation column 58 which is operated to provide a light overheads fraction including benzene which is supplied via line 59 to the alkylation reactor input and a heavier liquids fraction containing benzene, ethylbenzene and polyethylbenzenes.

The output from the prefractionation zone 58 is supplied via line 60 to a benzene separation zone 61. The overhead fraction from column 61 contains the remains benzene which is recycled via line 62 to the alkylation reactor input. The heavier bottoms fraction from column 61 is supplied via line 64 to an ethylbenzene separation zone 65. The overheads fraction from column 65, of course, comprises ethylbenzene which is supplied to storage or to any suitable product destination. By way of example, the ethylbenzene may be used as a feed stream to a styrene plant in which styrene is produced by the dehydrogenation of ethylbenzene. The bottoms fraction containing polyethylbenzenes, heavier aromatics and preferably only a small amount of ethylbenzene, no more than 5% as discussed previously, is supplied to polyethylbenzene separation zone 68. The bottoms fraction of column 68 comprises a residue. The overhead fraction from column 68, containing polyethylbenzene, triethylbenzene (usually in relatively small quantities) and a minor amount of ethylbenzene is supplied to a transalkylation reaction zone 72. By minimizing the amount of ethylbenzene recovered from the bottom of column 65, the ethylbenzene content of the transalkylation feed stream is kept small in order to drive the transalkylation reaction in the direction of ethylbenzene production. The polyethylbenzene fraction withdrawn overhead through line 70 is mixed with benzene supplied via line 73 and then supplied to the transalkylation reactor 72. The mol ratio of benzene to polyethylbenzenes should be at least 1:1 and preferably is within the range of 1:1 to 4:1. The output from the transalkylation reactor containing benzene, ethylbenzene and diminished amounts of polyethylbenzenes is supplied via line 75 to the benzene column 61.

In the process depicted in FIGURE 7, the alkylation reaction is carried out in the liquid phase with dehydration of feed to the alkylation reactor. As noted previously, the invention may be carried out employing vapor-phase alkylation followed by liquid phase transalkylation and in such reactions, depending upon the catalyst employed, significant quantities of water may be included in the feed to the alkylation reactor. In this case, it may be necessary to separately accomplish dehydration of the feed to the transalkylation reactor. Such dehydration may take place at any point upstream of the transalkylation reactor, and if necessary, dehydration should be accomplished with respect to the fresh benzene feed supplied via line 73 as well as with respect to the polyethylbenzne component produced during the alkylation reaction.

FIGURE 8 discloses a modification of the process disclosed in FIGURE 7 in which the transalkylation reactor output is subjected to further treatment prior to recycle to the separation system. The embodiment of FIGURE 8 is particularly useful in those cases in which relatively high conversion is achieved in the transalkylation reactor. In the embodiment of FIGURE 8, the alkylation reactor and separation system is identical to that of FIGURE 7 and like components are indicated by the same reference characters. However, the output from the transalkylation reactor is supplied to a secondary separation zone 77 which may take the form of a distillation column which is operated in a manner to produce a bottom purge stream withdrawn via line 78 and a recycle stream withdrawn via line 80 and supplied to the benzene column.

The purge stream containing heavy hydrocarbons is withdrawn from the system, thus providing a partially single pass system in which high molecular weight hydrocarbons are not recirculated.

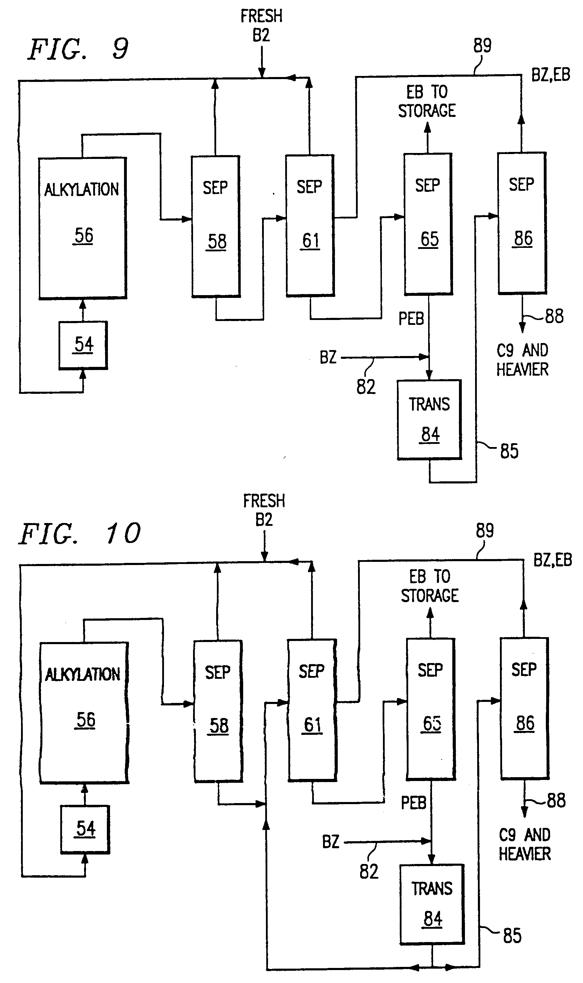

FIGURE 9 illustrates yet another embodiment of the invention in which the polyethylbenzene fraction recovered from the ethylbenzene column is directly passed to a transalkylation reactor. In FIGURE 9, the same system components as shown in FIGURES 7 and 8 are designated by like reference numerals. As shown in FIGURE 9, the output from the ethylbenzene column 65 is mixed with benzene supplied via line 82 and supplied to the transalkylation reactor 84. Here, the entire polyethylbenzene fraction is subjected to transalkylation. The conditions employed in reactor 84 may be the same as described above with the ratio of benzene to polyethylbenzenes ranging from about 1:1 to 4:1.

It will be recognized that the procedure depicted in FIGURE 9 is similar to that of FIGURE 8 except that the entire bottoms fraction from the ethylbenzene column is subjected to the transalkylation reaction. Limiting the ethylbenzene content of the input to the transalkylation reactor to no more than 5%, preferably 2% or less is especially significant here in establishing conditions promoting the transalkylation reaction. The output from the transalkylation reactor is applied via line 85 to a post transalkylation separation zone 86 which may take the form of a distillation column operated to produce an overhead fraction that is comprised predominantly of benzene and ethylbenzene and a bottoms fraction, composed predominantly of C₉ and C₁₀ hydrocarbons such as ethyltoluene, cumene, butylbenzene etc., which is eliminated from the recycle stream by purge line 88. The overheads fraction is recycled through line 89 to the benzene column similarly as described above.

The embodiment of FIGURE 10 is similar to that of FIGURE 9 except that the transalkylation reactor output is split, with a portion being directly supplied to the benzene column 61 via line 92 and the remainder to the separation zone 86 which is operated as described above. The configuration of FIGURE 10 provides a means for maintaining a low concentration of C₉ and C₁₀ hydrocarbons in the system and reduces the energy costs of operating column 86. Typically about 60% or more of the transalkylation reactor output is recycled directly to the benzene column 61 with the remainder being directed to the separation zone 86.

Having described specific embodiments of the present invention, it will be understood that modification thereof may be suggested to those skilled in the art, and it is intended to cover all such modifications as fall within the scope of the appended claims.

## Claims

1. In an alkylation-transalkylation process, the steps comprising :
a) supplying a feed stock containing an aromatic substrate into a reaction zone containing a molecular sieve aromatic alkylation catalyst;
b) supplying a C₂-C₄ alkylating agent to said reaction zone;
c) causing alkylation of said aromatic substrate by said alkylating agent in the presence of said catalyst to produce an alkylated product comprising a mixture of monoalkylated and polyalkylated aromatic products;
d) recovering said alkylated product from said reaction zone and supplying said product from said reaction zone to a separation zone for the separation of said aromatic substrate;
e) operating said separation zone to produce a lower boiling fraction comprising said aromatic substrate and a higher boiling fraction comprising a mixture of monoalkylated aromatic-polyalkylated aromatic mixture;
f) supplying said higher boiling fraction from said separation zone to a second separation zone;
g) operating said second separation zone to produce a second lower boiling fraction comprising monoalkylated aromatic product and a higher boiling fraction comprising heavier polyalkylated aromatic product;
h) supplying at least a portion of said polyalkylated aromatic product including substantially all of the dialkylated and trialkylated aromatics in said polyalkylated product to a transalkylation reaction zone containing a molecular sieve transalkylation catalyst;
i) supplying said aromatic substrate to said transalkylation zone;
j) operating said transalkylation reaction zone under temperature and pressure conditions to maintain said aromatic substrate in the liquid phase and effective to cause disproportionation of said polyalkylated aromatic fraction to arrive at a disproportionation product having a reduced polyalkylated aromatic content and an enhanced monoalkylated aromatic content; and
k) supplying at least a portion of said disproportionation product to said first recited separation zone.

2. Process according to Claim 1 wherein said aromatic substrate is benzene, said alkylating agent is an ethylating or propylating agent, and further comprising prior to step (k) in Claim 1, supplying the output from said transalkylation zone to a third separation zone and operating said third separation zone to produce a lower boiling fraction comprising a benzene-monoalkylbenzene component and a higher boiling fraction comprising a polyalkylbenzene component and supplying said benzene-monoalkylbenzene component to said first recited separation zone in accordance with step (k) of Claim 1.

3. Process according to any of the preceding claims wherein said alkylation reaction zone is operated under temperature and pressure conditions to cause vapor phase alkylation of said aromatic substrate.

4. Process according to Claim 3 wherein said aromatic substrate is benzene, said alkylating agent is ethylene and said transalkylation reaction zone is operated at an average temperature at least 100°C less than the average temperature of said alkylation reaction zone.

5. Process according to any of Claims 1 to 2 wherein the reaction zone at step (c) is operated at temperature and pressure conditions to maintain said aromatic substrate in the liquid phase.

6. Process according to Claim 1 wherein said aromatic substrate is benzene, said alkylating agent is an ethylating agent, said alkylation catalyst is a zeolite beta or a zeolite omega and wherein at step (g) said heavier polyalkylated aromatic product (polyethylbenzene) contains no more than 5 wt % monoalkylated aromatic product (ethylbenzene).

7. Process according to any of the preceding claims wherein the aromatic substrate is benzene.

8. Process according to any of the preceding claims wherein said alkylating agent is an olefin.

9. Process according to claim 8 wherein said olefin is ethylene.

10. Process according to any of the preceding claims wherein said aromatic alkylation catalyst comprises a molecular sieve selected from the group consisting of zeolite beta, zeolite omega and zeolite Y.

11. Process according to any of the preceding claims wherein said transalkylation catalyst comprises a molecular sieve selected from the group consisting of zeolite Y and zeolite omega.

12. Process according to claim 10 wherein zeolite beta is chosen.

13. Process according to claim 11 wherein zeolite omega is chosen.

14. Process according to claim 7 wherein said transalkylation zone is operated at a space velocity (LHSV) based upon benzene and alkyl benzenes which is less than the space velocity (LHSV) in said primary reaction zone based upon benzene.

15. Process according to claim 14 wherein said velocity in said transalkylation zone is within the range of 1-10 LHSV.

16. Process according to claim 15 wherein said transalkylation zone space velocity is less than one half the space velocity of said primary alkylation zone.

## Patentansprüche

1. In einem Alkylierungs-Transalkylierungs-Verfahren umfassen die Schritte:
a) Zuführen eines ein aromatisches Substrat enthaltenden Ausgangsmaterials in eine Reaktionszone, die einen aromatischen Alkylierungs-Katalysator mit einem Molekularsieb enthält;
b) Zuführen eines C₂-C₄-Alkylierungsmittels in die Reaktionszone;
c) Durchführen der Alkylierung des aromatischen Substrats mit dem Alkylierungsmittel in der Gegenwart des Katalysators, um ein alkyliertes Produkt herzustellen, das ein Gemisch von monoalkylierten und polyalkylierten aromatischen Produkten umfaßt;
d) Gewinnen des alkylierten Produktes aus der Reaktionszone und Zuführen des Produktes aus der Reaktionszone in eine Trennzone zur Abtrennung des aromatischen Substrats;
e) Betreiben der Trennzone zur Herstellung einer tiefersiedenden Fraktion, die das aromatische Substrat enthält, und einer höhersiedenden Fraktion, die ein Gemisch eines monoalkylierten aromatischen-polyalkylierten aromatischen Gemisches enthält;
f) Zuführen der höhersiedenden Fraktion aus der Trennzone in eine zweite Trennzone;
g) Betreiben der zweiten Trennzone zur Herstellung einer zweiten tiefersiedenden Fraktion, die ein monoalkyliertes aromatisches Produkt enthält, und einer höhersiedenden Fraktion, die ein schwereres polyalkyliertes aromatisches Produkt enthält;
h) Zuführen von mindestens einem Teil des polyalkylierten aromatischen Produkts, der im wesentlichen all die dialkylierten und trialkylierten Aromaten in dem polyalkylierten Produkt enthält, zu einer Transalkylierungsreaktionszone, die einen Molekularsieb-Transalkylierungskatalysator enthält;
i) Zuführen des aromatischen Substrats in die Transalkylierungszone;
j) Betreiben der Transalkylierungsreaktionszone unter Temperatur- und Druckbedingungen, um das aromatische Substrat in der flüssigen Phase zu halten, und die dazu wirksam sind, eine Disproportionierung der polyalkylierten aromatischen Fraktion zu bewirken, um ein Disproportionierungsprodukt mit einem verringerten Gehalt an polyalkylierten Aromaten und einem erhöhten Gehalt an monoalkylierten Aromaten zu erhalten; und
k) Zuführen von mindestens einem Teil des Disproportionierungsprodrnktes in die zuerst genannte Trennzone.

2. Verfahren nach Anspruch 1, wobei das aromatische Substrat Benzol ist, das Alkylierungsmittel ein Ethylierungs- oder Propylierungsmittel ist, und das außerdem vor dem Schritt (k) in Anspruch 1 das Zuführen des Ausstoßes der Transalkylierungszone in eine dritte Trennzone und Betreiben der dritten Trennzone zur Herstellung einer tiefersiedenden Fraktion, die eine Benzol-Monoalkylbenzol-Komponente umfaßt, und einer höhersiedenden Fraktion, die eine Polyalkylbenzol-Komponente umfaßt, und Zuführen der Benzol-Monoalkylbenzol-Komponente in die zuerst genannte Trennzone gemäß Schritt (k) von Anspruch 1 umfaßt.

3. Verfahren nach einem der vorstehenden Ansprüche, wobei die Alkylierungsreaktionszone bei Temperatur- und Druckbedingungen betrieben wird, die eine Dampfphasenalkylierung des aromatischen Substrats bewirken.

4. Verfahren nach Anspruch 3, wobei das aromatische Substrat Benzol ist, das Alkylierungsmittel Ethylen ist und die Transalkylierungsreaktionszone bei einer mindestens 100 °C unterhalb der durchschnittlichen Temperatur der Alkylierungsreaktionszone liegenden durchschnittlichen Temperatur betrieben wird.

5. Verfahren nach einem der Ansprüche 1 bis 2, wobei die Reaktionszone im Schritt (c) bei Temperatur- und Druckbedingungen betrieben wird, bei denen das aromatische Substrat in der flüssigen Phase gehalten wird.

6. Verfahren nach Anspruch 1, wobei das aromatische Substrat Benzol ist, das Alkylierungsmittel ein Ethylierungsmittel ist, der Alkylierungs-Katalysator ein Zeolith beta oder ein Zeolith omega ist und wobei im Schritt (g) das schwerere polyalkylierte aromatische Produkt (Polyethylbenzol) nicht mehr als 5 Gew.-% monoalkyliertes aromatisches Produkt (Ethylbenzol) enthält.

7. Verfahren nach einem der vorstehenden Ansprüche, wobei das aromatische Substrat Benzol ist.

8. Verfahren nach einem der vorstehenden Ansprüche, wobei das Alkylierungsmittel ein Olefin ist.

9. Verfahren nach Anspruch 8, wobei das Olefin Ethylen ist.

10. Verfahren nach einem der vorstehenden Ansprüche, wobei der aromatische Alkylierungs-Katalysator ein Molekularsieb enthält, ausgewählt aus der Gruppe, die aus Zeolith beta, Zeolith omega und Zeolith Y besteht.

11. Verfahren nach einem der vorstehenden Ansprüche, wobei der Transalkylierungs-Katalysator ein Molekularsieb umfaßt, ausgewählt aus der Gruppe, die aus Zeolith Y und Zeolith omega besteht.

12. Verfahren nach Anspruch 10, wobei Zeolith beta ausgewählt wird.

13. Verfahren nach Anspruch 11, wobei Zeolith omega ausgewählt wird.

14. Verfahren nach Anspruch 7, wobei die Transalkylierungszone bei einer auf Benzol und Alkylbenzole bezogenen Raumgeschwindigkeit (LHSV) betrieben wird, die geringer ist, als die auf Benzol bezogene Raumgeschwindigkeit (LHSV) in der primären Reaktionszone.

15. Verfahren nach Anspruch 14, wobei die Geschwindigkeit in der Transalkylierungszone in dem Bereich von 1 bis 10 LHSV liegt.

16. Verfahren nach Anspruch 15, wobei die Transalkylierungszonen-Raumgeschwindigkeit weniger als die Hälfte der Raumgeschwindigkeit der primären Alkylierungszone beträgt.

## Revendications

1. Procédé d'alkylation-transalkylation comprenant les étapes :
a) amener une charge contenant un substrat aromatique dans une zone de réaction contenant un catalyseur d'alkylation aromatique à tamis moléculaire;
b) amener un agent d'alkylation C₂-C₄ dans la dite zone de réaction;
c) provoquer l'alkylation du dit substrat aromatique par le dit agent d'alkylation en présence du dit catalyseur pour produire un produit alkylé comprenant un mélange de produits aromatiques monoalkylés et polyalkylés;
d) récupérer le dit produit alkylé de la dite zone de réaction et amener le dit produit de la dite zone de réaction dans une zone de séparation pour la séparation du dit substrat aromatique;
e) opérer la dite zone de séparation pour produire une fraction à bas point d'ébullition comprenant le dit substrat aromatique et une fraction à haut point d'ébullition comprenant un mélange de produit aromatique monoalkylé et de produit aromatique polyalkylé;
f) amener la dite fraction à haut point d'ébullition de la dite zone de séparation dans une seconde zone de séparation;
g) opérer la dite seconde zone de séparation pour produire une deuxième fraction à bas point d'ébullition comprenant le produit aromatique monoalkylé et une fraction à haut point d'ébullition comprenant le produit aromatique polyalkylé plus lourd;
h) amener au moins une partie du dit produit aromatique polyalkylé comprenant substantiellement tous les aromatiques dialkylés et trialkylés du dit produit polyalkylé dans une zone de réaction de transalkylation contenant un catalyseur de transalkylation à tamis moléculaire ;
i) amener le dit substrat aromatique dans la dite zone de transalkylation;
j) opérer la dite zone de réaction de transalkylation dans des conditions de température et de pression afin de maintenir le dit substrat aromatique en phase liquide et afin de provoquer la dismutation de la dite fraction aromatique polyalkylée pour arriver à un produit de dismutation ayant une teneur réduite en aromatique polyalkylée et une teneur accrue en aromatique monoalkylée; et
k) amener au moins une partie du dit produit de dismutation dans la dite zone de séparation citée en premier lieu.

2. Procédé selon la revendication 1 dans lequel le dit substrat aromatique est le benzène, le dit agent d'alkylation est un agent d'éthylation ou de propylation, et comprenant, en supplément, avant l'étape (k) de la revendication 1, amener ce qui sort de la dite zone de transalkylation dans une troisième zone de séparation et opérer la dite troisième zone de séparation pour produire une fraction à bas point d'ébullition comprenant un constituant benzène-monoalkylbenzène et une fraction à plus haut point d'ébullition comprenant un constituant polyalkylbenzène, et amener le dit constituant benzène-monoalkylbenzène dans la dite zone de séparation citée en premier lieu en accord avec l'étape (k) de la revendication 1.

3. Procédé selon une quelconque des revendications précédentes dans lequel la dite zone de réaction d'alkylation est opérée dans des conditions de température et de pression afin de provoquer l'alkylation en phase vapeur du dit substrat aromatique.

4. Procédé selon la revendication 3 dans lequel le dit substrat aromatique est le benzène, le dit agent d'alkylation est l'éthylène, et la dite zone de réaction de transalkylation est opérée à une température moyenne d'au moins 100°C inférieure à la température moyenne de la dite zone de réaction d'alkylation.

5. Procédé selon une quelconque des revendications 1 et 2 dans lequel la zone de réaction à l'étape (c) est opérée dans des conditions de température et de pression afin de maintenir le dit substrat aromatique en phase liquide.

6. Procédé selon la revendication 1 dans lequel le dit substrat aromatique est le benzène, le dit agent d'alkylation est un agent d'éthylation, le dit catalyseur d'alkylation est une zéolite béta ou une zéolite oméga, et dans lequel à l'étape (g) le dit produit aromatique polyalkylé plus lourd (polyéthylbenzène) ne contient pas plus de 5% en poids de produit aromatique monoalkylé (éthylbenzène).

7. Procédé selon l'une quelconque des revendications précédentes dans lequel le dit substrat aromatique est le benzène.

8. Procédé selon l'une quelconque des revendications précédentes dans lequel le dit agent d'alkylation est une oléfine.

9. Procédé selon la revendication 8 dans lequel la dite oléfine est l'éthylène.

10. Procédé selon l'une quelconque des revendications précédentes dans lequel le dit catalyseur d'alkylation aromatique comprend un tamis moléculaire choisi dans le groupe constitué des zéolite béta, zéolite oméga et zéolite Y.

11. Procédé selon l'une quelconque des revendications précédentes dans lequel le dit catalyseur de transalkylation comprend un tamis moléculaire choisi dans le groupe constitué des zéolite Y et zéolite oméga.

12. Procédé selon la revendication 10 dans lequel la zéolite béta est choisie.

13. Procédé selon la revendication 11 dans lequel la zéolite oméga est choisie.

14. Procédé selon la revendication 7 dans lequel la dit zone de transalkylation est opérée à une vitesse spatiale (LHSV), basée sur le benzène et les alkylbenzènes, qui est inférieure à la vitesse spatiale (LHSV), basée sur le benzène, de la dite première zone de réaction.

15. Procédé selon la revendication 14 dans lequel la dite vitesse dans la dite zone de transalkylation est dans la gamme de 1-10 LHSV.

16. Procédé selon la revendication 15 dans lequel la dite vitesse spatiale de zone de transalkylation est inférieure à la moitié de la vitesse spatiale de la dite première zone d'alkylation.
